# EUROPÄISCHE PATENTANMELDUNG

(11) **EP 1 430 908 A2**
(43) Veröffentlichungstag der Anmeldung: **23.06.2004**
(21) Anmeldenummer: 03029080.3
(22) Anmeldetag: 17.12.2003
(51) Int. Cl.: A61L 2/18, A61L 2/22, A61L 2/26, B65B 55/10, B67C 7/00, G01F 11/16

(54) **Sterilisiervorrichtung für Behälter**

(30) Priorität: 18.12.2002 DE 20219642 U
(71) Anmelder: Salda, Luciano, 41058 Vignola (MO) (IT)
(72) Erfinder: Salda, Luciano, 41058 Vignola (MO) (IT)
(74) Vertreter: Grünecker, Kinkeldey, Stockmair & Schwanhäusser Anwaltssozietät

(57) **Zusammenfassung**

Die vorliegende Erfindung bezieht sich auf eine Sterilisiervorrichtung (1) für Behälter (29), insbesondere für Flaschen. Die Sterilisiervorrichtung weist einen Vorratsbehälter (4) zur Aufnahme eines Sterilisiermittels auf, eine Zuleitung (3) für ein Trägerfluid, eine zwischen dem Vorratsbehälter (4) und der Zuleitung (3) vorgesehene Zuführöffnung (6) sowie eine Vorrichtung (2) zum Mischen von Trägerfluid und Sterilisiermittel. Die Mischvorrichtung (2) umfasst ein zumindest abschnittsweise durch die Zuführöffnung (6) bewegbares Dosierelement (7), das einen Hohlraum (31) aufweist und bewegbar ist zwischen einer Füllstellung, in der der Hohlraum (31) ausschließlich zum Vorratsbehälter (4) geöffnet ist, und einer Mischstellung, in der der Hohlraum (31) ausschließlich zur Zuleitung (3) geöffnet ist. Die erfindungsgemäße Sterilisiervorrichtung (1) zeichnet sich dadurch aus, dass die Mischvorrichtung (2) ferner eine als Doppelkammerpumpe ausgebildete Kolbenpumpe (13) mit einem in einem Gehäuse (12) geführten Kolben (15) aufweist, wobei in der Kolbenpumpe (13) eine Mischkammer (17) zur Aufnahme des Gemisches aus Trägerfluid und Sterilisiermittel und eine Druckkammer (18) vorgesehen sind.

## Beschreibung

Die vorliegende Erfindung betrifft eine Sterilisiervorrichtung gemäß dem Oberbegriff des Anspruches 1. Sie kann beispielsweise im medizinischen Bereich oder im Lebensmittelbereich eingesetzt werden, um Behälter zu sterilisieren, insbesondere Flaschen.

Eine Sterilisiervorrichtung ist aus der DE 198 08 318 A1 bekannt. Bei dieser bekannten Sterilisiervorrichtung wird Luft als Trägerfluid eingesetzt. Die Mischvorrichtung besteht in einer Vernebelungsdüse, mittels derer das Sterilisierungsmittel in den Luftstrom eingeblasen und darin vernebelt wird. Dieser Nebel wird schließlich in die zu sterilisierenden Behälter eingeleitet, um an deren Wänden einen Kondensatfilm zu bilden. Nachteilig an der bekannten Sterilisiervorrichtung ist, dass mit ihr das Mischungsverhältnis zwischen Trägerfluid und Sterilisierungsmittel nur ungenau eingestellt werden kann.

Eine weitere Sterilisiervorrichtung ist aus der EP 0 727 229 A2 bekannt. Sie hat einen Mischkolben, auf dem zwei ringförmige, als Nuten geformte Dosierkammern vorgesehen sind. Jeweils eine dieser Dosierkammern oder Mischungen wird über eine Passage mit Sterilisierfluid gefüllt, während die andere Dosierkammer ihren Inhalt in eine Druckluft-Passage ausstößt. Anschließend wird der Mischkolben so bewegt, dass die leere Dosierkammer neu befüllt wird, während die gefüllte Dosierkammer mit der Druckluftpassage in Verbindung gebracht wird.

Der Aufbau der aus der EP 0 727 229 bekannten Sterilisiervorrichtung ist verhältnismäßig kompliziert, da zwei Mischnuten auf dem Kolben vorgesehen sein müssen und mehrere Verzweigungen der Druckluftleitungen notwendig sind. Zudem ist nicht gewährleistet, dass das Sterilisierfluid und die Druckluft am Ausgang der Vorrichtung den größtmöglichen Durchmischungsgrad erreicht haben.

Aufgabe der vorliegenden Erfindung ist es daher, eine Sterilisiervorrichtung zur Verfügung zu stellen, die trotz eines einfachen Aufbaus die Einstellung des Mischungsverhältnisses deutlich verbessert und vor allem einen hohen Durchmischungsgrad gewährleistet.

Diese Aufgabe wird gelöst durch eine Sterilisiervorrichtung mit den Merkmalen des Anspruches 1.

Das Einstellen eines genauen Mischungsverhältnisses wird durch das Dosierelement erreicht, in dessen Hohlraum in der Füllstellung eine definierte Menge an Sterilisiermittel aufgenommen wird, die anschließend in der Mischstellung an das durch die Zuleitung fließende Trägerfluid abgegeben wird. Die dosierte Menge des Sterilisiermittels wird durch die Größe des Hohlraumes vorgegeben. Das Dosierelement wirkt bei der erfindungsgemäßen Sterilisiervorrichtung als eine Art Schleuse, die das Sterilisiermittel über den Hohlraum zur Zuleitung transportiert, ohne dass die Zuführöffnung vom Vorratsbehälter zur Zuleitung jemals durchgängig für das Sterilisiermittel geöffnet ist. Die Zuführung des Sterilisiermittels findet daher sehr kontrolliert statt.

Der Vorteil der Erfindung liegt darin, dass in der Mischvorrichtung zusätzlich eine als Doppelkammerpumpe ausgebildete Kolbenpumpe verwendet wird. Die Mischkammer bietet dabei zum einen einen Sammelraum, in dem eine bestimmte Menge des Trägerfluid-Sterilisiermittel-Gemisches gesammelt wird, bevor es durch die Kraft des Kolbens ausgestoßen und zu dem zu sterilisierenden Behälter bewegt wird. Zum anderen bietet die Mischkammer einen Ruheraum, in dem das Gemisch aus Trägerfluid und Sterilisiermittel verweilen und dabei seinen Durchmischungsgrad maximieren kann.

Die Druckdifferenz zwischen den beiden Kammern bestimmt dabei auf einfache Weise die Bewegung des Kolbens und entscheidet insbesondere darüber, wann und wie schnell das Ausstoßen des Gemisches aus der Mischkammer durchgeführt wird.

In einer bevorzugten Ausführungsform dichtet das Dosierelement die Zuführöffnung ab, um den freien Ausfluss von Sterilisiermittel in die Zuleitung zu verhindern.

Günstig ist es, wenn das Dosierelement in seiner Mischstellung wenigstens teilweise in die Zuleitung hinein vorsteht. Dadurch bilden sich im Trägerfluid Verwirbelungen, die die Mitnahme des Sterilisiermittels aus dem Hohlraum begünstigen. Zudem erhöht sich durch den verringerten Querschnitt der Zuleitung die Fließgeschwindigkeit des Trägerfluids, und es bietet sich die Möglichkeit, den Hohlraum unter Umständen vollständig im Inneren der Zuleitung anzuordnen.

Das Dosierelement selbst kann beispielsweise als Kolbenstange ausgebildet sein. In einer einfachen Ausführungsform ist diese Kolbenstange zylindrisch, und die Zuführöffnung hat einen kreisförmigen Durchmesser, der an denjenigen der Kolbenstange angepasst ist.

Zweckmäßig ist es, wenn der Hohlraum zwei Seitenöffnungen aufweist. Dies erleichtert die Mitnahme des Sterilisiermittels durch das Trägerfluid, da das Trägerfluid in der Mischstellung durch den Hohlraum hindurch strömen kann.

Sehr einfach herzustellen ist solch ein Hohlraum mit zwei Seitenöffnungen, wenn er als durchgehende Querbohrung durch das Dosierelement ausgebildet ist. Durch diese Querbohrung kann das Trägerfluid zudem besonders gut hindurchströmen. Daher genügt eine geringe Verweildauer des Dosierelements in der Füllstellung, bis das im Hohlraum enthaltene Sterilisiermittel vollständig vom Trägerfluid mitgenommen wurde.

Die zur Mitnahme des Sterilisiermittels benötigte Zeit kann noch weiter verringert werden, indem die Querbohrung in der Mischstellung etwa parallel zu einer Strömungsrichtung des Trägerfluids in der Zuleitung angeordnet ist. Zudem kann der Hohlraum in der Mischstellung zu dem gleichen Zweck etwa in der Mitte des Querschnittes der Zuleitung angeordnet sein, da dort bei einer laminaren Strömung des Trägerfluids die höchste Strömungsgeschwindigkeit vorliegt.

Als Antrieb für das Dosierelement stehen verschiedene Möglichkeiten zur Verfügung. Bevorzugt wird ein pneumatischer Antrieb für das Dosierelement verwendet.

Um zu verhindern, dass beim Ausstoßen des Sterilisiergemisches aus der Mischkammer ein Rückfluß in die Zuleitung stattfindet, kann ein Rückschlagventil vorgesehen sein. Es sollte insbesondere zwischen der Zuführöffnung und der Mischkammer angeordnet sein.

Um den Druckausgleich zwischen den Kammern zu verhindern, ist es zweckmäßig, wenn die Mischkammer und die Druckkammer mittels des Kolbens gegeneinander abgedichtet sind.

In einer eleganten Ausführungsform der Erfindung ist die Druckkammer von der Zuleitung aus mit Trägerfluid füllbar. Auf diese Weise erfüllt das Trägerfluid zwei verschiedene Aufgaben: zum einen dient es als Trägermaterial für das Sterilisiermittel, zum anderen dient es zum Aufbau des Drucks in der Druckkammer, wodurch schließlich das in der Mischkammer befindliche Trägerfluid-Sterilisiermittel-Gemisch ausgestoßen wird.

Das Trägerfluid kann bereits vorgeheizt sein. Es ist jedoch vorteilhaft, wenn auch die Mischkammer der Kolbenpumpe beheizbar ist. So kann das sich in ihr ansammelnde Sterilisiergemisch auf eine gewünschte Temperatur gebracht werden, die insbesondere so hoch ist, dass das Gemisch später an dem zu sterilisierenden Behälter kondensiert.

In einer bevorzugten Ausführungsform weist das Gehäuse der Kolbenpumpe wenigstens abschnittsweise einen Doppelmantel auf, durch den zum Beheizen der Mischkammer ein Heizmittel führbar ist. Auf diese Weise kann eine große Oberfläche zum Aufheizen der Mischkammer ausgenutzt werden; idealerweise sogar die gesamte Oberfläche der Mischkammer. Der Doppelmantel sollte dazu selbstverständlich aus einem wärmeleitenden Material bestehen.

Um erkaltendes Heizmittel abzuführen, kann am Doppelmantel ein Kondensatstauer vorgesehen sein. Ist er regelbar, so kann über ihn die Temperatur des Gemisches in der Mischkammer eingestellt werden.

Ideal ist es, wenn das Trägerfluid selbst als Heizmittel verwendet wird. Damit entfällt die Notwendigkeit, ein zusätzliches Heizmittel zur Verfügung stellen und in einem separaten Kreislauf behandeln zu müssen. Die Konstruktion der erfindungsgemäßen Sterilisiervorrichtung vereinfacht sich erheblich.

Vorzugsweise umfaßt das Trägerfluid zu mindestens 50% Wasserdampf, vorzugsweise von 90% bis 100%. Gegenüber der bisher verwendeten Luft als Trägerfluid hat Wasserdampf eine enorm verbesserte Wärmekapazität, während es ebenso günstig erhältlich und ebenso leicht handhabbar ist. Wasserdampf kann daher die verschiedenen Aufgaben als Verdünnungs- und Transportmittel für das Sterilisiermittel, als Mittel zum Druckaufbau und als Heizmittel besonders gut erfüllen.

Um den Betrieb der erfindungsgemäßen Sterilisiervorrichtung zu vereinfachen, kann die Vorrichtung einen Taktgeber aufweisen, mittels dessen das Dosierelement mit derselben Frequenz wie die Kolbenpumpe oder einem ganzzahligem Vielfachen dieser Frequenz betreibbar ist. Damit wird ausgenutzt, dass sowohl das Dosierelement, als auch die Kolbenpumpe intervallartig arbeiten, und die beiden Arbeitszyklen werden aufeinander abgestimmt. So kann sichergestellt werden, dass in jeder vollständigen Füllung der Mischkammer genau einmal die durch den Hohlraum des Dosierelementes vorgegebene Menge an Sterilisiermittel enthalten ist. Wird eine stärkere Konzentration benötigt, so kann das Dosierelement zweimal, dreimal oder noch öfter in jedem Arbeitszyklus der Kolbenpumpe betrieben werden. In jeder Füllung der Mischkammer ist dann die doppelte, dreifache oder allgemein n-fache Konzentration an Sterilisiermittel gegeben. Der Taktgeber kann zudem mit nachfolgenden Komponenten der Sterilisiervorrichtung zusammenwirken. Beispielsweise kann er mit einem Träger für die zu sterilisierenden Behälter so zusammenwirken, dass die Aufnahme eines neuen Behälters auf dem Träger und das Ausstoßen des Sterilisier-Gemisches aus der Kolbenpumpe zeitlich aufeinander abgestimmt werden.

Im Folgenden wird ein Ausführungsbeispiel der Erfindung anhand einer Zeichnung beschrieben. Im Einzelnen zeigen:
- Figur 1: einen schematisch dargestellten Ausschnitt eines Ausführungsbeispiels einer erfindungsgemäßen Sterilisiervorrichtung, wobei sich der Kolben der Kolbenpumpe in seiner Plus-Position befindet,
- Figur 2: die in Figur 1 gezeigte Darstellung mit dem Kolben der Kolbenpumpe in seiner Minus-Position,
- Figur 3: einen Vertikalschnitt durch das Dosierelement in dessen Füllstellung, und
- Figur 4: einen Vertikalschnitt durch das Dosierelement in dessen Mischstellung.

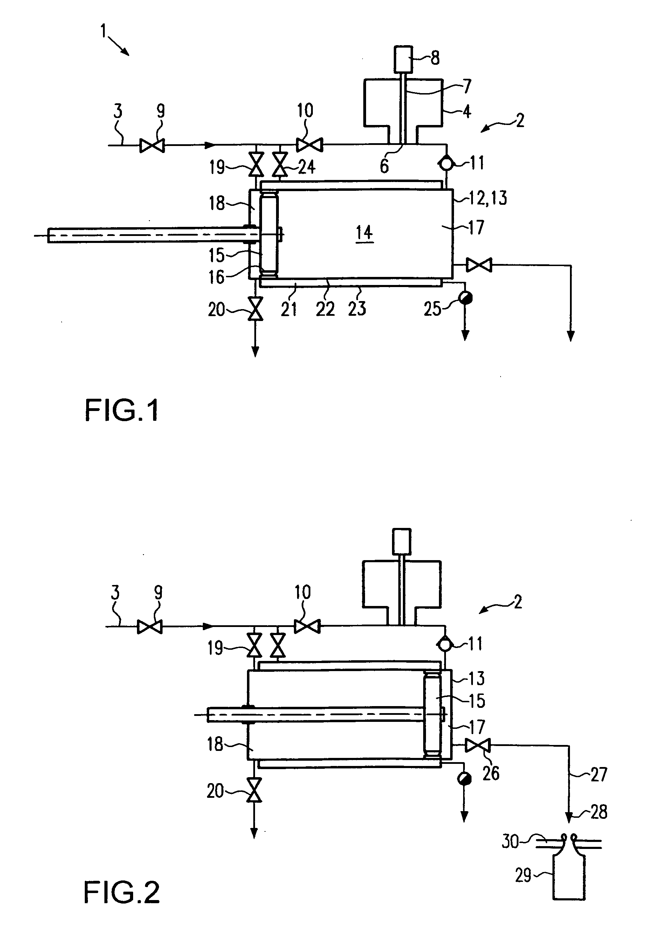

Figur 1 stellt schematisch einen Teil eines Ausführungsbeispiels einer erfindungsgemäßen Sterilisiervorrichtung 1 dar, insbesondere eine Mischvorrichtung 2, mit der das zum Sterilisieren verwendete Sterilisiergemisch erzeugt wird. Das Sterilisiergemisch wird gebildet aus einem Trägerfluid, das über eine Zuleitung 3 in die Mischvorrichtung 2 eingeleitet wird, und einem Sterilisiermittel, das in einem Vorratsbehälter 4 gespeichert wird. In dem dargestellten Ausführungsbeispiel wird als Trägerfluid reiner Wasserdampf eingesetzt, der außerhalb der dargestellten Mischvorrichtung 2 erzeugt wird. Die Zuleitung 3 ist als Rohr mit einem konstanten Durchmesser ausgebildet.

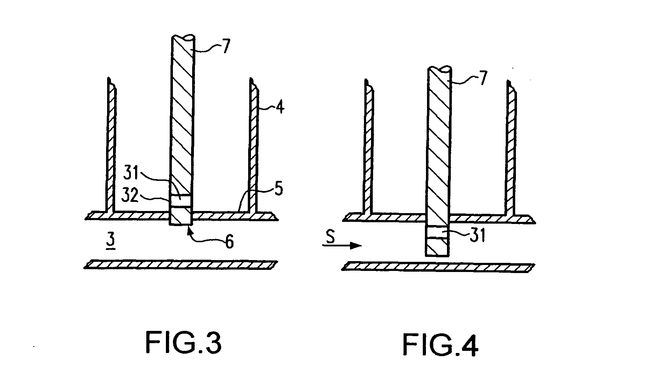

Der Vorratsbehälter 4 liegt unmittelbar an der Zuleitung 3 an. In der gemeinsamen Wand 5 zwischen Zuleitung 3 und Vorratsbehälter 4 befindet sich eine Zuführöffnung 6 (siehe Figuren 3, 4). Ein Dosierelement 7, das über einen pneumatischen Antrieb 8 verfügt und dessen Funktionsweise anhand der Figuren 3 und 4 näher beschrieben wird, führt Sterilisiermittel aus dem Vorratsbehälter 4 in die Zuleitung 3.

In Strömungsrichtung S des Trägerfluids befinden sich in der Zuleitung 3 ein erstes Ventil 9, ein zweites Ventil 10, die Zuführöffnung 6 und ein Rückschlagventil 11, bevor die Zuleitung 3 in das Gehäuse 12 einer Kolbenpumpe 13 einmündet. Das Gehäuse 12 der Kolbenpumpe 13 umschließt einen zylindrischen Innenraum 14. In dem Innenraum 14 ist als Kolben eine kreisförmige Kolbenscheibe 15 angeordnet. Entlang ihres Umfanges verfügt sie über ein Abdichtelement 16, das sie ringförmig gegen die Wände des Innenraumes 14 gasdicht abdichtet. Auf diese Weise teilt die Kolbenscheibe 15 den Innenraum 14 in zwei Kammern: eine Mischkammer 17 und eine Druckkammer 18. Folglich ist die Kolbenpumpe 13 eine Doppelkammerpumpe. Das jeweilige Volumen der Mischkammer 17 und der Druckkammer 18 wird durch die momentane Stellung der in Axialrichtung des Innenraumes 14 bewegbaren Kolbenscheibe 15 vorgegeben.

Die Zuleitung 3 mündet jenseits des Rückschlagventils 11 so in die Kolbenpumpe 13, dass sie mit der Mischkammer 17 in Fluidverbindung steht. Das durch die Zuleitung 3 heranströmende Gemisch aus Trägerfluid und Sterilisiermittel strömt daher in die Mischkammer 17 ein. Der sich in der Mischkammer 17 aufbauende Druck führt zu einer Bewegung der Kolbenscheibe 15, so dass sich das Volumen der Mischkammer 17 vergrößert und dasjenige der Druckkammer 18 verringert. In der in Figur 1 gezeigten Stellung befindet sich die Kolbenscheibe 15 in ihrer Plus-Stellung, in der die Mischkammer 17 ihr maximales und die Druckkammer 18 ihr minimales Volumen annehmen.

Die Druckkammer 18 kann mittels eines dritten Ventils 19 von der Zuleitung 3 aus mit Trägerfluid befüllt werden. Auf diese Weise kann in der Druckkammer 18 ein Druck aufgebaut werden, um die Kolbenscheibe 15 von der Plus- in ihre Minus-Position zu bewegen. Aus der Druckkammer 18 kann das darin enthaltene Trägerfluid über ein viertes Ventil 20 abgelassen werden.

Über nahezu seine gesamte Länge weist das Gehäuse 12 der Kolbenpumpe 13 einen Doppelmantel 21 mit einem Innenmantel 22 und einem Außenmantel 23 auf. Zumindest der Innenmantel 22, der die Wand des Innenraumes 14 bildet, besteht aus einem wärmeleitenden Material. Über ein fünftes Ventil 24 ist der Doppelmantel 21 von der Zuleitung 3 aus mit Trägerfluid befüllbar. Da hier Wasserdampf als Trägerfluid verwendet wird, kann das Trägerfluid im Doppelmantel 21 als Heizmittel für das Sterilisiergemisch in der Mischkammer 17 dienen. Auf diese Weise wird ein Temperaturverlust wettgemacht, den das Trägerfluid auf dem weiteren Weg durch die Zuleitung 3 zur Mischkammer 17 und insbesondere durch das Einleiten von Sterilisiermittel erlitten hat. Das erkaltete Heizmittel (hier: Trägerfluid) wird aus dem Doppelmantel 21 über einen Kondensatstauer 25 abgeführt. Ein Temperatursensor (nicht dargestellt) am Doppelmantel 21 oder in der Mischkammer 17 kann das fünfte Ventil 24 so regeln, dass dieses neues, heißes Trägerfluid in den Doppelmantel 21 einströmen läßt, wenn die Temperatur unter einen vorgegebenen Wert gesunken ist. So wird gewährleistet, dass das in der Mischkammer 17 enthaltene Sterilisiergemisch eine bestimmte Temperatur einhält.

Figur 2 zeigt dieselbe Mischvorrichtung 2, wie sie bereits anhand von Figur 1 beschrieben wurde. Im Unterschied zur Figur 1 befindet sich die Kolbenscheibe 15 der Kolbenpumpe 13 nun in ihrer Minus-Position. Dabei nehmen die Mischkammer 17 ihr minimales und die Druckkammer 18 ihr maximales Volumen an. Das Gemisch aus Trägerfluid und Sterilisiermittel, das in der Mischkammer 17 enthalten war, ist durch die Bewegung der Kolbenscheibe 15 über ein sechstes Ventil 26 in eine Ableitung 27 abgegeben worden. Die Ableitung 27 mündet in einen Einblaspunkt 28, an dem das Sterilisiergemisch in einen zu sterilisierenden Behälter 29 eingeblasen wird. Es ist angedeutet, dass der Behälter 29 von einem Greifer 30 gehalten wird, der ein Teil eines Transportsterns sein kann.

Die Figuren 3 und 4 zeigen als Ausschnitt aus der Mischvorrichtung 2 das Dosierelement 7. Es ist als zylindrische Kolbenstange ausgebildet, wobei sich als Material Edelstahl besonders gut eignet. Das Dosierelement 7 ragt durch die Zuführöffnung 6 in der Wand 5 zur Zuleitung 3 hindurch. Die Zuführöffnung 6 hat einen kreisförmigen Durchmesser, der gerade ausreicht, um das Dosierelement 7 darin aufzunehmen. Die Zuführöffnung 6 wird daher durch das Dosierelement 7 abgedichtet.

An einem Endabschnitt des Dosierelementes 7 ist eine durchgehende Querbohrung vorgesehen, die zwei Seitenöffnungen 32 aufweist und im Dosierelement 7 einen Hohlraum 31 bildet.

In Figur 3 befindet sich das Dosierelement 7 in seiner Füllstellung, in der der Hohlraum 31 vollständig innerhalb des Vorratsbehälters 4 angeordnet ist. Der Hohlraum 31 steht dabei über die Seitenöffnungen 32 ausschließlich mit dem Inneren des Vorratsbehälters 4 in Fluidkontakt, so dass das im Vorratsbehälter 4 enthaltene Sterilisiermittel in den Hohlraum 31 einströmt. Das Volumen des Hohlraumes 31 gibt dabei vor, welche Menge an Sterilisiermittel vom Dosierelement 7 aufgenommen wird.

Ist der Hohlraum 31 vollständig mit Sterilisiermittel gefüllt, so bewegt der pneumatische Antrieb 8 das Dosierelement 7 von der Füllstellung in die in Figur 4 gezeigte Mischstellung. Im dargestellten Ausführungsbeispiel wird dazu das als Kolbenstange ausgebildete Dosierelement 7 in seiner Längsrichtung durch die Zuführöffnung 6 in die Zuleitung 3 hineinbewegt. In der Mischstellung befindet sich der Hohlraum 31 vollständig innerhalb der Zuleitung 3. Er ist so ausgerichtet, dass seine Längsachse parallel zur Strömungsrichtung S des heranströmenden Trägerfluids liegt. Das Trägerfluid strömt daher durch den Hohlraum 31 hindurch und reißt das darin enthaltene Sterilisiermittel mit sich mit. Auf der Luv-Seite des Dosierelementes 7 bildet sich ein Gemisch aus dem Trägerfluid und dem Sterilisiermittel. Da das in die Zuleitung 3 hineinragende Ende des Dosierelementes 7 eine Störung darstellt, bilden sich im Bereich der Zuführöffnung 6 Verwirbelungen, die eine Durchmischung von Trägerfluid und Sterilisiermittel begünstigen. Sobald das im Hohlraum 31 enthaltene Sterilisiermittel vollständig vom Trägerfluid mitgerissen wurde, kann das Dosierelement mittels seines Antriebes 8 wieder in die Füllstellung zurückgefahren werden.

Im Folgenden wird die Funktionsweise des dargestellten Ausführungsbeispiels der erfindungsgemäßen Sterilisiervorrichtung 1 beschrieben. Ausgangspunkt ist die in Figur 2 gezeigte Stellung der Mischvorrichtung 2. In dieser Stellung werden das dritte Ventil 19 und das sechste Ventil 26 geschlossen, während das zweite Ventil 10 und das vierte Ventil 20 geöffnet werden. Damit wird der Weg für eine Befüllung der Mischkammer 17 über die Zuleitung 3 geöffnet, während sich die Druckkammer 18 über das vierte Ventil 20 entleeren kann.

In der Füllstellung des Dosierelementes 7 füllt sich dessen Hohlraum 31 mit Sterilisiermittel. Sobald der Hohlraum 31 vollständig befüllt ist, wird das Dosierelement 7 mittels seines Antriebes 8 in die Mischstellung verfahren. Das über die ersten und zweiten Ventile 9, 10 durch die Zuleitung 3 heranströmende Trägerfluid reißt das Sterilisiermittel aus dem Hohlraum 31 mit sich fort. In der Zuleitung 3 bildet sich ein Sterilisiergemisch aus Trägerfluid und Sterilisiermittel, das in der Strömungsrichtung S über das Rückschlagventil 11 in die Mischkammer 17 der Kolbenpumpe 13 einströmt. Nach dem Entleeren des Hohlraumes 31 fährt das Dosierelement 7 in seine Füllstellung zurück.

Das Sterilisiergemisch aus Trägerfluid und Sterilisiermittel füllt die Mischkammer 17. Da das sechste Ventil 26 geschlossen ist, baut sich in der Mischkammer 17 ein Druck auf. Die Druckkammer 18 bietet wegen des geöffneten vierten Ventiles 20 keinen Widerstand, so dass die Druckdifferenz zwischen Mischkammer 17 und Druckkammer 18 zu einer Verschiebung der Kolbenscheibe 15 führt. Das Volumen der Mischkammer 17 vergrößert sich, während das Volumen der Druckkammer 18 abnimmt. Durch das heiße Trägerfluid im Doppelmantel 21 werden der Innenmantel 22 und darüber das in der Mischkammer 17 enthaltene Sterilisiergemisch beheizt. Die Mischkammer 17 dient somit erstens zum Ansammeln eines bestimmten Volumens des Sterilisiergemisches, zweitens zum Verweilen des Sterilisiergemisches, um dessen Vermischungsgrad zu erhöhen, und drittens zum Beheizen des Sterilisiergemisches.

Der Befüllungsvorgang der Mischkammer 17 ist abgeschlossen, wenn die Kolbenscheibe 15 ihre in Figur 1 gezeigte Plus-Position erreicht hat. In dieser Stellung hat die Mischkammer 17 ihr maximales Volumen. Die Plus-Position der Kolbenscheibe 15 könnte über einen Positionssensor festgestellt werden. Ist diese Plus-Stellung erreicht, so werden das zweite Ventil 10 und das vierte Ventil 20 geschlossen. Das dritte Ventil 19 wird geöffnet, so dass von der Zuleitung 3 Trägerfluid in die Druckkammer 18 hineinströmen kann. Dadurch baut sich in der Druckkammer 18 ein Druck auf. Das sechste Ventil 26 wird geöffnet, so dass das in der Mischkammer 17 enthaltene Gemisch über die Ableitung 27 zum Einblaspunkt 28 abgeführt wird, da sich die Kolbenscheibe 15 aufgrund des Druckes in der Druckkammer 18 in ihre Minus-Position bewegt. Der Zeitpunkt, zu dem das sechste Ventil 26 geöffnet wird, bestimmt dabei, mit welcher Heftigkeit das Sterilisiergemisch ausgestoßen wird. Wird das sechste Ventil 26 gleichzeitig mit dem dritten Ventil 19 geöffnet, so erfolgt der Ausstoß relativ langsam. Wird das sechste Ventil 26 erst zu einem späteren Zeitpunkt geöffnet, so hat sich in der Druckkammer 18 bereits ein gewisser Druck aufgebaut. Daher erfolgen die Bewegungen der Kolbenscheibe 15 und der Ausstoß des Sterilisiergemisches schneller. Das Rückschlagventil 11 verhindert, dass das Sterilisiergemisch aus der Mischkammer 17 in die Zuleitung 3 zurückströmt. Der einzige Weg aus der Mischkammer 17 führt über die Ableitung 27 zum Einblaspunkt 28 und in den zu sterilisierenden Behälter 29. Dieser wird auf einer niedrigeren Temperatur gehalten als das erhitzte Sterilisiergemisch. Daher kondensiert das Sterilisiergemisch am Behälter 29 und bildet einen sterilisierenden Kondensatfilm.

An diesem Punkt ist ein Zyklus der intervallartig arbeitenden Mischvorrichtung 2 beendet. Ein neuer Zyklus kann durch das Schließen des dritten und des sechsten Ventiles 19, 26 sowie durch das Öffnen des zweiten und vierten Ventiles 10, 20 eingeleitet werden.

In der beschriebenen Betriebsweise findet pro Zyklus der Kolbenpumpe 13 lediglich ein Zyklus des Dosierelementes 7 statt. Die beiden Komponenten werden also mit derselben Frequenz betrieben, und in einer Füllung der Mischkammer 17 befindet sich je einmal die durch den Hohlraum 31 vorgegebene Dosierung an Sterilisiermittel. Wird eine höhere Konzentration benötigt, so kann das Dosierelement 7 zweimal, dreimal oder allgemein n-mal pro Arbeitszyklus der Kolbenpumpe betrieben werden. Damit gelangt die doppelte, dreifache oder n-fache Dosierung an Sterilisiermittel in eine Füllung der Mischkammer 17. Da die Konzentration des Sterilisiermittels im Sterilisiergemisch ganzzahlig variiert wird, kann diese Konzentration sehr genau auf den gewünschten Wert eingestellt werden. Um die Arbeitszyklen des Dosierelementes 7 und der Kolbenpumpe 13 in ihrer Frequenz aufeinander abzustimmen, weist die Sterilisiervorrichtung einen nicht dargestellten Taktgeber auf. Der von ihm vorgegebene Takt ist an den Arbeitszyklus der Transportvorrichtung für die zu sterilisierenden Behälter 29 angepasst.

Von dem beschriebenen Ausführungsbeispiel kann die erfindungsgemäße Sterilisiervorrichtung in vielfacher Hinsicht abgewandelt werden. In einer sehr simplen Ausführungsform könnte beispielsweise auf die Kolbenpumpe 13 verzichtet werden. In dieser Variante könnte das Dosierelement 7 immer kurz vor demjenigen Zeitpunkt in seine Mischstellung gebracht werden, zu dem Sterilisiergemisch in einen neuen Behälter 29 eingeblasen werden soll. So wird kurz vor dem Einblasen die Konzentration des Sterilisiermittels im Sterilisiergemisch erhöht.

Das Dosierelement 7 muß nicht notwendigerweise als Kolbenstange ausgebildet sein, solange seine Querschnittsform an diejenige der Zuführöffnungen 6 angepasst bleibt. Elegant wäre ein Dosierelement 7, bei dem das Volumen des Hohlraumes 31 variabel ist. Damit könnte eine variable, insbesondere stufenlose Dosierung des Sterilisiermittels vorgenommen werden.

Das Beheizen der Mischkammer 17 muß nicht unbedingt über das Trägerfluid erfolgen. Stattdessen könnte ein anderes Heizmittel oder auch eine elektrische Beheizung vorgesehen sein. Schließlich könnte das Trägerfluid auch zum Betreiben des Antriebes 8 des Dosierelementes 7 eingesetzt werden.

## Patentansprüche

1. Sterilisiervorrichtung (1) für Behälter (29), insbesondere Flaschen, mit einem Vorratsbehälter (4) zur Aufnahme eines Sterilisiermittels, einer Zuleitung (3) für ein Trägerfluid, einer zwischen dem Vorratsbehälter (4) und der Zuleitung (3) vorgesehenen Zuführöffnung (6) und einer Vorrichtung (2) zum Mischen von Trägerfluid und Sterilisiermittel, wobei die Mischvorrichtung (2) ein zumindest abschnittsweise durch die Zuführöffnung (6) bewegbares Dosierelement (7) umfasst, das einen Hohlraum (31) aufweist und bewegbar ist zwischen einer Füllstellung, in der der Hohlraum (31) ausschließlich zum Vorratsbehälter (4) geöffnet ist, und einer Mischstellung, in der der Hohlraum (31) ausschließlich zur Zuleitung (3) geöffnet ist,
**dadurch gekennzeichnet, dass** die Mischvorrichtung (2) ferner eine als Doppelkammerpumpe ausgebildete Kolbenpumpe (13) mit einem in einem Gehäuse (12) geführten Kolben (15) aufweist, wobei in der Kolbenpumpe (13) eine Mischkammer (17) zur Aufnahme des Gemisches aus Trägerfluid und Sterilisiermittel und eine Druckkammer (18) vorgesehen sind.

2. Sterilisiervorrichtung nach Anspruch 1, **dadurch gekennzeichnet, dass** das Dosierelement (7) die Zuführöffnung (6) abdichtet.

3. Sterilisiervorrichtung nach Anspruch 2, **dadurch gekennzeichnet, dass** das Dosierelement (7) in der Mischstellung wenigstens teilweise in die Zuleitung (3) hinein vorsteht.

4. Sterilisiervorrichtung nach einem der vorangehenden Ansprüche, **dadurch gekennzeichnet, dass** das Dosierelement (7) als Kolbenstange ausgebildet ist.

5. Sterilisiervorrichtung nach Anspruch 4, **dadurch gekennzeichnet, dass** die Kolbenstange (7) zylindrisch ist und die Zuführöffnung (6) einen kreisförmigen Durchmesser hat.

6. Sterilisiervorrichtung nach wenigstens einem der vorangehenden Ansprüche, **dadurch gekennzeichnet, dass** der Hohlraum (31) zwei Seitenöffnungen (32) aufweist.

7. Sterilisiervorrichtung nach wenigstens Anspruch 6, **dadurch gekennzeichnet, dass** der Hohlraum (31) als durchgehende Querbohrung durch das Dosierelement (7) ausgebildet ist.

8. Sterilisiervorrichtung nach wenigstens Anspruch 7, **dadurch gekennzeichnet, dass** die Querbohrung (31) in der Mischstellung etwa parallel zu einer Strömungsrichtung (S) des Trägerfluids in der Zuleitung (3) angeordnet ist.

9. Sterilisiervorrichtung nach wenigstens einem der vorangehenden Ansprüche, **dadurch gekennzeichnet, dass** der Hohlraum (31) in der Mischstellung etwa in der Mitte des Querschnittes der Zuleitung (3) angeordnet ist.

10. Sterilisiervorrichtung nach wenigstens einem der vorangehenden Ansprüche, **dadurch gekennzeichnet, dass** das Dosierelement (7) einen pneumatischen Antrieb (8) aufweist.

11. Sterilisiervorrichtung nach wenigstens einem der vorangehenden Ansprüche, **dadurch gekennzeichnet, dass** zwischen der Zuführöffnung (6) und der Mischkammer (17) der Kolbenpumpe (13) ein Rückschlagventil (11) vorgesehen ist.

12. Sterilisiervorrichtung nach wenigstens einem der vorangehenden Ansprüche, **dadurch gekennzeichnet, dass** die Mischkammer (17) und die Druckkammer (18) mittels des Kolbens (15) gegeneinander abgedichtet sind.

13. Sterilisiervorrichtung nach wenigstens einem der vorangehenden, **dadurch gekennzeichnet, dass** die Druckkammer (18) von der Zuleitung (3) aus mit Trägerfluid füllbar ist.

14. Sterilisiervorrichtung nach wenigstens einem der vorangehenden Ansprüche, **dadurch gekennzeichnet, dass** die Mischkammer (17) der Kolbenpumpe (13) beheizbar ist.

15. Sterilisiervorrichtung nach wenigstens einem der vorangehenden Ansprüche, **dadurch gekennzeichnet, dass** das Gehäuse (12) der Kolbenpumpe (13) wenigstens abschnittsweise einen Doppelmantel (21) aufweist, durch den zum Beheizen der Mischkammer (17) ein Heizmittel führbar ist.

16. Sterilisiervorrichtung nach wenigstens Anspruch 15, **dadurch gekennzeichnet, dass** das Heizmittel über einen Kondensatstauer (25) aus dem Doppelmantel (21) ableitbar ist.

17. Sterilisiervorrichtung nach wenigstens einem der Ansprüche 14 bis 16, **dadurch gekennzeichnet, dass** das Trägerfluid als Heizmittel verwendet wird.

18. Sterilisiervorrichtung nach wenigstens einem der vorangehenden Ansprüche, **dadurch gekennzeichnet, dass** das Trägerfluid zu mindestens 50%, vorzugsweise von 90% bis 100%, Wasserdampf umfasst.

19. Sterilisiervorrichtung nach wenigstens einem der vorangehenden Ansprüche, **dadurch gekennzeichnet, dass** sie einen Taktgeber aufweist, mittels dessen das Dosierelement (7) mit derselben Frequenz wie die Kolbenpumpe (13) oder einem ganzzahligen Vielfachen dieser Frequenz betreibbar ist.
